(19) Europäisches Patentamt / European Patent Office / Office européen des brevets

(11) **EP 2 294 222 B1**

(12) **EUROPEAN PATENT SPECIFICATION**

(45) Date of publication and mention
of the grant of the patent:
**27.04.2016 Bulletin 2016/17**

(51) Int Cl.:
**C12Q 1/68** (2006.01)

(21) Application number: **09772842.2**

(22) Date of filing: **01.07.2009**

(86) International application number:
**PCT/GB2009/050765**

(87) International publication number:
**WO 2010/001162 (07.01.2010 Gazette 2010/01)**

(54) **FREEZE-DRIED COMPOSITIONS FOR PCR AND RT-PCR**

GEFRIERGETROCKNETE ZUSAMMENSETZUNGEN FÜR PCR UND RT-PCR

COMPOSITIONS LYOPHILISÉES POUR DES RÉACTIONS PCR ET RT-PCR

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **02.07.2008 GB 0812041**

(43) Date of publication of application:
**16.03.2011 Bulletin 2011/11**

(73) Proprietor: **Enigma Diagnostics Ltd.
Salisbury, Wiltshire SP4 0JQ (GB)**

(72) Inventors:
• **FRANKS, Felix**
  **London N3 2PG (GB)**
• **SPIERS, Samantha**
  **Salisbury Wiltshire SP4 0JQ (GB)**
• **LEE, Martin**
  **Salisbury Wiltshire SP4 0JQ (GB)**
• **SUTTON, Diane**
  **Winchester Hampshire SO22 5DG (GB)**

(74) Representative: **Turner, Rhiannon Rosalind
Greaves Brewster LLP
Copa House
Station Road
Cheddar, BS27 3AH (GB)**

(56) References cited:
WO-A-2005/108620    WO-A-2008/075072
WO-A-2008/155524    US-A1- 2002 173 016

• BECKER N S C ET AL: "Detection of polyelectrolytes at trace levels in water by fluorescent tagging" REACTIVE & FUNCTIONAL POLYMERS, ELSEVIER SCIENCE PUBLISHERS BV, NL, vol. 60, 1 July 2004 (2004-07-01), pages 183-193, XP004518376 ISSN: 1381-5148
• DAVIDSON PAUL ET AL: "Effect of sucrose/raffinose mass ratios on the stability of co-lyophilized protein during storage above the Tg" PHARMACEUTICAL RESEARCH (NEW YORK), vol. 18, no. 4, April 2001 (2001-04), pages 474-479, XP002547014 ISSN: 0724-8741
• RIISOM T ET AL: "EFFECT OF AMINO-ACIDS ON THE AUT OXIDATION OF SAFFLOWER OIL IN EMULSIONS" JOURNAL OF THE AMERICAN OIL CHEMISTS' SOCIETY, vol. 57, no. 10, 1980, pages 354-359, XP002547015 ISSN: 0003-021X

**Description**

The present invention relates to compositions comprising test reagents for use in chemical or biochemical reactions such as the polymerase chain reaction and to methods for preparing these.

Freeze drying is a commonly used method for the long term storage of materials and in particular biological materials such as cells and proteins. However, many materials including proteins are unstable in this process. The problem has been addressed to a certain extent by the addition of sugars such as disaccharides such as sucrose, maltose, trehalose and lactose to the formulations for freeze drying. These sugars act as glass-forming agents but also and appear to protect proteins during the freeze drying process. It has been shown that this is due to the direct interaction of the proteins with the disaccharides through hydrogen bonding that is essential for the stabilisation effect as this prevents conformational changes of the protein during the freeze drying process.

This has meant that freeze drying can now be used to stabilise complex reaction systems including proteins such as enzymes and cells, in particular for storage purposes. Generally however, additional stabilisers such as polymers including polymeric compounds such as polyethylene glycol (PEG), polyvinylpyrrolidine (PVP) and or polysaccharides such as Ficoll or Dextran are used to maintain enzyme activity. These polymers are normally cake stabilizing excipients used to help form a uniform cake structure. This aids in the drying process by allowing a uniform matrix to form that allows efficient exit of water during sublimation. They therefore affect the final cake structure.

Further complications arise in the preparation of freeze dried mixtures of reagents which are routinely used together, for example in a chemical or biochemical reaction. For instance, the widely used polymerase chain reaction (PCR) (including reverse transcriptase polymerase chain reaction (RT-PCR) utilises a range of standard reagents including salts such as magnesium chloride ($MgCl_2$) and potassium chloride, a polymerase enzyme such as *Taq* polymerase, buffers such as Tris-HCl, and nucleotides required for an amplification of a nucleic acid. Such preparations are available for example as "ready-to-go PCR beads" from Amersham BioSciences (UK)/Pharmacia.

Generally these are prepared by freeze drying methods, which are conducted in the presence of glass-forming agents and stabilisers for the structures formed as well as optionally fillers (see for example US Patent No. 5,250,429, US Patent No. 5,763,157 and EP-0726310). Trehalose in particular has been used in PCR mixtures to assist in the stabilisation and also in non freeze dried formulations as a PCR enhancer. However, care needs to be taken in the selection of reagents used as glass-forming agents and stabilisers in these reaction mixtures to ensure that they do not interfere with or unduly inhibit the chemical or biochemical reaction in which the compositions are required to take part.

Nevertheless such beads provide a convenient and readily available means for laboratories to conduct PCR reactions of their choice, when required. Generally, the specific reagents which tailor a PCR to the particular target, such as the primers and any probes required for example for use in connection with a Real-time PCR, are added in addition to the core bead, "on site" as the reaction is prepared.

However, in many cases, in particular in the diagnostics field, the targets are the same in many cases, and therefore the inclusion of probes and primers into the bead, so that the bead becomes assay specific is desirable for ease of use.

By adding further reaction components in this way, the stability problem is increased as there are more components which are required to be maintained in stable form. As the nature of assays becomes more complex, the risk that other components in the reaction such as glass-forming agents and stabilisers will interfere with or inhibit the reaction increases.

Furthermore, the further reaction components may include reagents which may include relatively sensitive chemical moieties such as labels and in particular optical labels such as fluorescent labels or dyes. These in particular are used for conducting assays in "real-time". The sensitive moieties are frequently attached to oligonucleotides which may be designed to act as probes or labelled primers. These will hybridise to amplified nucleic acids during the course of the PCR. The fate of the probes during the course of the PCR and changes in the associated signal from the label is used in various ways to monitor the progress of the PCR.

However, the presence of such moieties can exacerbate the problems associated with the formation of the compositions, since the stabilising additives in compositions may result in a reduction or inhibition of the signalling function in particular from fluorescent labels.

Raffinose has previously been used as a glass-forming agent in the freeze-drying of cells or proteins as well as pharmaceutical preparations (Kajuwara et al. Pharmaceutical Research Vol 16, 9 1999, but has not hitherto been used in the freeze-drying of complex chemical or biochemical reaction mixtures. In such mixtures, as mentioned above, all components including glass forming agents must be selected carefully so that they produce stable and long lasting dried compositions, and do not inhibit or take part in the subsequent chemical reaction in any detrimental way.

WO2006/119280 describes lyophilised pellets for use in reactions such as the PCR, which include cryoprotectants. A wide range of cryoprotectants are listed in this reference including mixtures of raffinose with one or more 'polyalcohols' which appears in this context to be used, as it sometimes is, in the sense that it refers to sugar alcohols rather than any polyol, since the examples provided are mannitol or sorbitol. However only trehalose is demonstrated as being useful.

US2002/173016 relates to high throughput lyophilized polymerase devices and methods. WO2008/075072 relates to use of a stabilised freeze dried formulation in a biosensor. WO2005/108620 discloses methods and systems for processing polynucleotides such as DNA. Becket et al. (Reactive & Functional Polymers (2004) vol. 60 p 183-193) discusses detection of polyelectrolytes in water by fluorescence tagging. Davidson & Sun (Pharm. Res. (2001) vol. 18 p 474-79) is a discussion of the effect of sucrose/raffinose mass ratios on the stability of co-lyophilized protein during storage. Riisom et al. (JAOCA (1980) vol. 57 p 354-359) discusses the effect of amino acids on the autoxidation of safflower oil in emulsions.

The applicants have found a means of providing improved freeze-dried compositions for carrying out chemical or biochemical reactions, including those that utilise fluorescent labels.

The present application discloses the use of raffinose as a glass-forming agent for freeze-dried compositions intended for use in conducting chemical or biochemical reactions such as the polymerase chain reaction (PCR) or reverse transcriptase (RT) PCR. The applicants have found that raffinose acts as an effective glass forming agent and stabiliser for these compositions and is compatible with chemical or biochemical reactions such as PCR. The raffinose is suitably not mixed with monomeric polyalcohols or sugar alcohols such as mannitol or sorbitol, and neither are these additional compounds required in the composition of the invention. When used in this way, the raffinose does not inhibit the reaction, even in the case of complex 'real-time' PCR reactions. As a result, the use of additional stabilisers may be avoided and so inhibition of for example fluorescent signalling systems is reduced. Thus the present invention provides a composition for carrying out a a polymerase chain reaction (PCR) or a reverse transcriptase polymerase chain reaction (RT-PCR) that may be monitored in real-time, said composition being in a freeze-dried form and comprising (i) a set of reagents comprising at least some of the chemical or biochemical reagents necessary for conducting said PCR or RT-PCR, including a polymerase capable of extending a primer when adhered to a template nucleic acid sequence during the PCR, (ii) at least one fluorescent reagent used to monitor the progress of the PCR or RT-PCR in real time and (iii) raffinose, wherein the raffinose is present in an amount such that in a final composition, reconstituted from said composition in order to carry out a chemical or biochemical reaction, it is present in an amount of from 2.5-10%(m/v).

Raffinose (which may be in the form of raffinose pentahydrate) acts as an effective glass-forming agent, which is compatible with the PCR or RT-PCR.

Furthermore, raffinose has been found to produce more stable cakes than conventional sugars such as trehalose. This may be due to the fact that it is also able to act as a desiccant. The cakes obtained by freeze drying raffinose compositions may have better structure and handling properties as compared to trehalose containing compositions. In some instances, the results obtainable with raffinose are superior to those obtained with trehalose containing compositions as illustrated hereinafter.

Raffinose must be present in a glass-forming amount in the composition. However, it should not be present in such a significant amount that upon reconstitution of the composition by addition of water to produce a "final composition" for use in a chemical or biochemical reaction, it is present in such an amount that it inhibits or otherwise restricts the reaction. As will be discussed in more detail below, the composition prepared for drying (i.e the 'cake composition') may suitably be more dilute and therefore the weight percentage (by volume) of raffinose in the compositions prepared initially may be correspondingly lower. In the Examples provided below, formulations are presented as a list of the volumes of individual reagents (at a given concentration or specific activity). Where the volume is referred to as 'cake volume', this represents the volume of reagent that was used in the freeze drying process. Where the volume is referred to as a reaction or final volume this is the volume or concentration of the reagent in the PCR/RT-PCR reaction.

The applicants have found however that even when present at 10% m/v or 10% w/w, raffinose does not inhibit real-time PCR assays.

Compositions prepared in accordance with the invention have good stability and cake-forming properties. Complex reactions such as PCR and in particular real-time PCR are not significantly inhibited, even where fluorescent signalling occurs.

The compositions suitably further comprise an anti-oxidant and/or anti-maillard reagent. The applicants have found that threonine works as a particularly effective anti-oxidant and/or anti-maillard agent, and enhances the stability of the freeze-dried composition. In particular, L-threonine is used. Without being bound by theory, the threonine appears to react with any oxygen produced and therefore assist in the stabilisation of the mixture.

Furthermore, it has been found that the presence of threonine may stabilise the signalling achievable from fluorescent labels included in the composition, in particular when stored at elevated temperatures.

The amount of threonine in the composition will vary depending upon the precise nature of the composition. It is suitably selected so that it does not affect the pH of the composition, which may be important in some chemical or biochemical reactions. Typically however, it may be present in the composition in an amount of from 2-10mM, for example at about 2.5mM.

When a composition is freeze dried in the presence of a glass-forming reagent, it generally forms a "cake" type 3-dimensional structure. This structure is optionally supported by the inclusion of a suitable stabiliser for the cake structure, and so this is a further component of the mixture.

Examples of suitable stabilisers that may be included in the composition include polymeric compounds such as polyethylene glycol (PEG), polyvinylpyrrolidine (PVP) and or polysaccharides such as Ficoll or Dextran. In a particular embodiment, however, the stabiliser is omitted from the composition as it has been found that compounds such as PEG may contribute to the inhibition of fluorescent signals. When raffinose is used as the glass-forming agent, the applicants have found that the need for such compounds is reduced.

In some cases also, gelatine may be used to add stability to the cake. Gelatine may be obtained from a variety of sources including bovine (such as cow), porcine (such as pig), seaweed (carrageenan) or fish gelatine. (Any bovine material used is suitably from a certified BSE free source.) Fish gelatine in particular may be a preferred gelatine component.

Suitable fluorescent reagents include fluorescent dyes or intercalators such as SYBR®Green I, SYBR®Gold, ethidium bromide, YOPRO-1, and the SYTO dyes including green dyes such as SYTO 9 and red SYTO dyes such as SYTO® 17, SYTO® 59, SYTO® 60, SYTO® 61, SYTO® 62, SYTO® 63 and SYTO® 64.

They may also include oligonucleotides which act as probes or primers and are labelled with fluorescent labels. Suitable labels include fluorescein or fluorescein derivatives such as carboxyfluorescein compounds , such as 5-carboxyfluorescein, 6-carboxyfluorescein, or their succinimidyl esters, cyanine dyes or rhodamine dyes. Particular examples of such dyes include fluorescein, JOE, FAM, HEX, TET, TAMRA, ROX Cy5, Cy3, Cy5.5,BoDIPY FL, rhodamine, rhodamine green, rhodamine red, Oregon Green 488, 500 or 514, Texas red, LightCycler Red 610, 640, 670 or 705. Other such dyes include IDT Dyes MAX550, TEX615, TYE563, TYE665 and TYE705.

Dark quenchers may also be present. These are generally used in assay systems to modify fluorescent signals but without emitting detectable signals themselves. These are essentially nonfluorescent dyes include in particular azo dyes (such as DABCYL or DABSYL dyes and their structural analogs), triarylmethane dyes such as malachite green or phenol red, 4',5-diether substituted fluoresceins (as described for example in U.S. Patent No. 4,318,846, asymmetric cyanine dye quenchers (as described for example in WO 99/37717) and methyl red.

In particular, quenching moiety is DABCYL (4-(dimethylaminoazo) benzene-4-carboxylic acid) or a derivative thereof, such as the halide or amide derivative, which facilitates attachment of the moiety to an amino acid of an oligonucleotide.

In another embodiment, the quenching moiety is an essentially nonfluorescent derivative of 3- and/or 6-amino xanthene that is substituted at one or more amino nitrogen atoms by an aromatic or heteroaromatic ring system (for example as described in US Patent No. 6,399,392). These quenching dyes typically have absorption a maximum above 530 nm, have little or no observable fluorescence and efficiently quench a broad spectrum of luminescent emission, such as is emitted by chemilumiphores, phosphors, or fluorophores. In one embodiment, the quenching dye is a substituted rhodamine. In another embodiment, the quenching compound is a substituted rhodol.

There are several types of polymerase which can undergo primer extension;

1. DNA dependant DNA polymerases utilise DNA as the template to generate complementary DNA. *Taq* (From *Thermus aquaticus*) is an example and this is a thermostable *pol* enzyme. Its normal function is to repair breaks and mismatches in cellular (bacterial) DNA.

2. RNA dependant DNA polymerases utilise RNA as the template and will generate complementary DNA in a process called reverse transcriptase (RT). *MMULV* and *AMV* are examples of these enzymes. Generally the commonly known RNA dependant DNA polymerases are non-thermostable. Some DNA dependant DNA polymerases such as *Taq* do exhibit a very small amount of RNA dependant DNA polymerase activity, but this too low and is therefore of little use to any currently known application.

3. RNA/DNA dependant DNA polymerases are enzymes that under certain conditions may use either DNA and/or RNA as templates in primer extension reactions to produce DNA. *Tth* (from *Thermus thermophilus*) is a common example of this type of enzyme which is also thermostable, but there are others such as the *Tsec* enzyme available from Genesys UK Limited. A key requirement for most of these RNA polymerase's activities is the presence of manganese as the bivalent metal ion rather than magnesium (as used by most other enzymes).

Any of the above mentioned polymerase types or combinations of these may be used in the compositions of the invention, and the selection will be made on the basis of the intended purpose of the composition.

Reverse transcriptase PCR (RT-PCR) is process by which RNA is first converted into DNA before being exponentially amplified in a PCR cycle. This can happen in three different reaction scenarios:

1. Two-step RT-PCR where an RNA dependant DNA polymerase or an RNA/DNA dependant DNA polymerase is utilised in a first reaction. This reaction may be primed using specific primers, random primers, or even poly dT to amplify polyadenylated cell messages. An aliquot is then transferred to separate PCR reaction for exponential amplification.

2. Two-Step "combined" RT-PCR is where a RNA dependent DNA polymerase is blended with a DNA dependant DNA polymerase in the same reaction vessel. Specific primers are used but the process is in fact, two discrete processes (with respect to RNA and the DNA amplification). After the completion of the first reaction in which RNA is converted into DNA, the non-thermostable RNA dependant DNA polymerase is denatured during the first high temperature step of the PCR stage of thermal cycling.

3. One-Step RT-PCR uses a DNA/RNA dependant DNA polymerase so that both stages of the process occurs concomitantly in the same reaction vessel. RNA and DNA may be generated in all stages of primer extension throughout the PCR. This has the added advantage that a single thermostable enzyme is utilised throughout the process. Therefore, it is often the preferred approach in diagnostics simplifying formulations for production and automation of the overall amplification steps.

However, one-step RT-PCR is technically more challenging than normal PCR as the magnesium is replaced by manganese to stimulate sufficient RT activity. This has the following effects that make finding reaction optima more challenging:

1. Magnesium affects DNA binding kinetics. It helps DNA duplex formation by reducing charge repulsion in the Watson-Crick B-DNA double helix structure. The concentration range of manganese is much lower for PCR optima than that which may favour the DNA binding.
2. Manganese at high concentrations causes the DNA *pol* activity to misincorporate nucleotides, thus the fidelity of PCR maybe reduced at concentrations which favour primer and template binding.
3. Manganese requires that a Tris buffer (used in all other types of PCR buffer) be replaced with a bicine buffer.

The combination of the above factors increases the complexity and optimisation processes for a given one-step RT-PCR process. The chemistry is distinct from normal PCR and two-step RT-PCR process in both formulation and kinetics. Many groups have tried to address these differences to overcome and consolidate the chemistries. For example US patent 7179590 describes the application of a novel mutation in *Tth* polymerase that allows the polymerase RT activity to be stimulated by magnesium ions.

In a particular embodiment, where the composition is intended for RT-PCR, the polymerase used is the *Tsec* (GeneSys Ltd, UK) RNA/DNA dependant DNA polymerase. As illustrated hereinafter in Example 7, which may be lyophilised in an environmentally stable format in the composition of the invention using raffinose.

Suitably the set of reagents of item (i) above further comprises a buffer, salt (such as a magnesium and/or manganese salt, depending upon the requirements of the polymerase as outlined above), one or more primers and nucleotides required to construct the extension to the primer(s) which are required to effect a polymerase chain reaction to amplify a target DNA sequence. In a typical PCR, the buffers used will generally be such that the pH is between 7 and 9, for instance between 8.5 and 8.8 for instance between 8.0 and 8.8. However, it is possible that one or more of these elements may be missing in particular where these elements can be readily added later, for example in a rehydration buffer used to reconstitute the dried composition ready for use. In particular, the necessary salts may be added in this way and so the set of reagents of (i) may omit the salts. Where this is done, the composition may be supplied in the form of a kit with rehydration buffer, containing the necessary salt supplements.

Alternatively, the salts such as magnesium may be present but in concentrations which are lower than are required for use in the reaction, for example at concentrations of less than $500\mu$M, may be included in the composition. As described in WO2006/003439, it has been found that such small amounts of magnesium salts may in fact be beneficial to the stability of the composition.

The composition may further comprise a labelled oligonucleotide, such as a fluorescently labelled oligonucleotide(s) useful in monitoring the progress of a polymerase chain reaction in real time. Also as used herein, the expression "real-time" means that the polymerase chain reaction can be monitored as it progresses and without halting or opening the reaction vessel. By monitoring how the amplification occurs and in particular at which cycles exponential increase in amplicon becomes significant allows the amount of target nucleic acid present in the sample being subject to the PCR to be quantitated as is well known and understood in the art.

The amounts of the various components included in the composition will vary depending upon factors such as the precise nature of the particular component; the nature of the PCR which it is intended should be conducted etc. However, this will be determinable in each case using established protocols and procedures as would be understood in the art.

Suitable labelled oligonucleotides are any of the labelled probes or labelled primers which may be used in the monitoring of polymerase chain reactions in real time. Thus in a particular embodiment they will comprise probes which are capable of hybridising to the amplified nucleic acid sequence and which carry labels in particular, optical labels such as fluorescent labels which provide a signal which varies in accordance with the progress of the PCR.

Thus for probes intended to be utilised in a Taqman ® assay, for example, they will generally comprise a probe which carries two labels, one of which is able to act as a donor of energy and particularly fluorescent energy, and one of which is able to act as an acceptor of that energy or "quencher". Whilst the probe is intact, these labels are held in close proximity to each other so that interaction of energy occurs. In the case of fluorescent labels, this is known as flurorescent energy transfer (FET) or fluorescent resonant energy transfer (FRET).

The probes are designed to bind to a specific region on one strand of a template nucleic acid. Following annealing of the PCR primer to this strand, *Taq* enzyme extends the DNA with 5' to 3' polymerase activity. *Taq* enzyme also exhibits 5' to 3' exonuclease activity. Taqman ® probes are protected at the 3' end by phosphorylation to prevent them from priming *Taq* extension. If the Taqman ®probe is hybridised to the product strand, an extending *Taq* molecule will hydrolyse the probe, liberating the donor from acceptor. This means that the interaction between the donor and the acceptor is broken, so the signal from each, changes, and this change can be used as the basis of detection. The signal in this instance is cumulative, the concentration of free donor and acceptor molecules increasing with each cycle of the amplification reaction.

Hybridisation probes are available in a number of forms and these may also be included in the compositions. Molecular beacons are oligonucleotides that have complementary 5' and 3' sequences such that they form hairpin loops. Terminal fluorescent labels are in close proximity for FRET to occur when the hairpin structure is formed. Following hybridisation of molecular beacons to a complementary sequence the fluorescent labels are separated, so FRET does not occur, and this forms the basis of detection during a polymerase chain reaction.

Pairs of labelled oligonucleotides may also be used as probes in the detection of a polymerase chain reaction. These hybridise in close proximity on a PCR product strand-bringing donor and acceptor molecules together so that FRET can occur. Enhanced FRET is the basis of detection. Variants of this type include using a labelled amplification primer with a single adjacent probe.

WO 99/28500 describes a very successful assay for detecting the presence of a target nucleic acid sequence in a sample. In this method, a DNA duplex binding agent and a probe specific for said target sequence, is added to the sample. The probe comprises a reactive molecule able to absorb fluorescence from or donate fluorescent energy to said DNA duplex binding agent. This mixture is then subjected to an amplification reaction in which target nucleic acid is amplified, and conditions are induced either during or after the amplification process in which the probe hybridises to the target sequence. Fluorescence from said sample is monitored.

Thus, compositions adapted for use in this assay, known as "Resonsense"™ may also be prepared. In this instance, the composition will suitably further comprises a DNA duplex binding agent such as an intercalating dye.

An alternative form of this assay, which utilises a DNA duplex binding agent which can absorb fluorescent energy from the fluorescent label on the probe but which does not emit visible light, is described in WO2004/033726.

In general, all probes used in these types of assays are blocked to extension at the 3'end for example by phosphorylation, or by having a label directly attached at the 3' hydroxyl group. This prevents the probe from acting as a secondary primer, and being extended during the PCR, and so eliminates interfering products. Alternatively one label may be positioned such that the label sterically inhibits extension by the enzyme rather than being chemically inhibited, and/or the 3' base or the probe may contain a mismatch such that the efficiency of extension is dramatically reduced as is well understood in the art.

The amounts of probe utilized in any particular composition will vary depending upon factors such as whether it is consumed or hydrolysed during the PCR, as well as the nature of the signalling system. These would be understood by the skilled person. Generally however, the amount of the or each probe added to a composition will be sufficient to ensure that the concentration of probe in the final composition is between $0.05\mu M$ to $1\mu M$, for example at about $0.2\mu M$.

Other real-time assays utilize labelled primers in order to provide a monitoring system. Some of these primers may include a self-probing "tail" and are known as "Scorpion" primers. A labelled probe is linked to a DNA sequence which acts as a primer to the reaction by way of a "blocking group" which is suitably a chemical linker or non-amplifiable monomer such as hexethylene glycol and which prevents an extension reaction amplifying the probe region of the oligonucleotide. Probe/primer combinations of this general type are well known as "Scorpions" and these are described for instance in WO 99/66071. The Scorpion may along its length comprise a donor/quencher pair so that FRET signalling is possible as described above.

A further class of real-time probes called LUX™ (light upon extension) fluorogenic primers are also available. These are "hairpin" like probes, similar to the molecular beacons as described above. However, LUX primers adopt a stem-loop structure in solution, and like Scorpion probes, LUX primers are intended for use as PCR primers. They do not contain a quencher moiety as they are fluorescent oligonucleotides which are designed to self-quench based on sequence context. LUX primers quench when free in solution, fluoresce weakly when denatured, and emit light strongly when incorporated into DNA. These also may be included in the compositions of the invention.

The polymerase included in the set of reagents (i) is selected so that it is useful in conducting the desired "real-time" assay. Thus for assays such as Taqman ®, where hydrolysis of the probe is essential in order to initiate a detectable signal, a polymerase having a high level of 5'-3' exonuclease activity is suitably employed, whereas for assays such as Resonsense™ assays, where probe hybridization is employed, such activity may be low or absent. The polymerase is suitably a thermostable polymerase which will operate and withstand the elevated temperatures needed for conducting a polymerase chain reaction. The amount of polymerase added should be sufficient to effect a PCR reaction, as is understood in the art. Typically, the amount of polymerase added will be sufficient to provide a final concentration of from 0.02 to 1.0U/μl reaction composition and typically about 0.025U/μl.

Suitably, the reaction composition may further comprise reagents which are used in ensuring that the polymerase chain reaction does not start prematurely. So called "Hot-Start" PCR may be effected by various methods.

The problem addressed by a "Hot-Start" PCR arises because a successful PCR relies on the sequence of steps, denaturation, annealing and extension, occurring in a very precise order and at the precise temperature required for the operation of that step. A problem arises when reagents are mixed together, even for short periods of time, at different temperatures, for example prior to the start of the reaction. Primers may interact with nucleic acid template, resulting in primer extension of the template. This can lead to a reduction in the overall yield of the desired product as well as the production of non-specific products.

Initial attempts to overcome the problem used a wax barrier to separate the various PCR reagents from each other in a test tube (see for example USP 5,565,339). The wax melted as the reaction mixture was heated to the initial denaturation temperature, allowing the reagents to mix together at the last possible moment, so that the possibility of side-reactions was minimized, and this gave rise to the expression "Hot Start".

Other chemical methods for achieving the suppression of side-reactions have been attempted. For example, US Patent No. 5,677,152 describes a method in which the DNA polymerase is chemically modified to ensure that it only becomes active at elevated temperatures. In order to effect this method, it is necessary only to include an appropriately modified DNA polymerase in item (i) above.

In another embodiment, a monoclonal antibody to *Thermus aquaticus* (*Taq*) DNA polymerase such as the *anti-Taq* DNA polymerase antibody available from Clontech, Sigma & Invitrogen, is including into the composition. The antibody binds to the enzyme active site, so as to inactivate it, at ambient temperature. However, the antibody denatures and dissociates from the enzyme at elevated temperatures used during the amplification cycles and so the enzyme becomes active.

The relative amount of any anti-*Taq* antibody included in the composition is suitably sufficient to ensure that it is able to fulfil the function of inhibiting the *Taq* enzyme until it is required. Generally therefore an excess of anti-Taq antibody as compared to *Taq* enzyme will be used. Thus for example for every unit of *Taq* enzyme in the composition, at least 1.5 and preferably at least 2 units of anti-Taq antibody will be included. *Taq* antibody is usually sold by the μg and the concentration is very dependant upon the source and quality of the antibody as well as the nature of the assay. Too much antibody may be detrimental and can actually cause more primer dimer in some assays. However, the precise amount of *Taq* antibody will be determined in accordance with usual practice and will typically be in the range of 0.001 to 0.004 μg/μl final reaction mixture.

Yet another Hot-Start methodology involving the use of a combination of an inhibitory amount of a pyrophosphate salt to prevent primer extension taking place, and a pyrophosphatase enzyme which digests this pyrophosphate at elevated temperatures, to allow the PCR to progress is described in WO 02/088387.

In this case, the pyrophosphate salt and the pyrophosphatase enzyme may be included as further components of the composition of the invention.

The use and precise selection of optional stabiliser will depend to some extent on the particular assay intended to be carried out using the final composition and this can be tested using routine methods. For example, it has been found that dextran is less preferred when the composition includes DNA duplex binding agents and labelled probes intended and is intended to be used to conduct a ResonSense™ assay as described above. However, PEG is a stabiliser that may be tolerated for most of these compositions. Stabiliser, where used, is suitably added in an amount such that it represents from about 1-3%m/v or1-3%w/w in the final composition.

As discussed above, the set of reagents of item (i) may comprise components such as buffers, primers, nucleotides and optionally also salts, in the amounts which are generally understood for the preparation of PCR reaction mixtures. Primers are suitably present in excess and this is typically achieved by including sufficient primers to ensure that the concentration of each primer in the final reaction composition is of the order of 0.1 μM to 1μM. As discussed in more detail below however, the compositions prepared as a cake composition for drying are suitably more dilute, and thus the molar concentration of primers (and indeed all other reagents) in these compositions will be correspondingly lower.

In a particular embodiment, a blocking compound, as is conventional in PCR reaction mixtures, may be included in the composition. The blocking compound is believed to function by preventing inhibition of the PCR by interaction with the vessel walls, for example by preventing leaching of metals or sequestering any metals which may leach from the walls in the course of the reaction. It may also reduce abstraction of enzyme and nucleotides to the reaction vessel wall. The nature of the blocking compound will depend upon the nature of the vessel into which it is intended that the reaction should be conducted.

Particular examples of blocking compounds are glass coating or glass blocking compounds such as bovine serum albumin (BSA) either alone or in combination with other blocking materials such as gelatine. As described above, gelatine may be obtained from a variety of sources including bovine, pig, seaweed (carrageenan) or fish gelatine.

Blocking agents are suitably included in effective amounts which will depending upon the particular compound selected. However, for BSA for instance, the amount is suitably sufficient to provide from 0.1 to 1mg/ml and preferably about 0.25mg/ml in the final reaction composition (i.e the composition made up for carrying out the chemical or biochemical reaction). Gelatines will suitably be present in an amount in the range of from about 0.0025%-0.01%m/v or about 0.0025%-0.01%w/w. Care should be taken that the amount of blocking agent is not sufficiently high so as to significantly inhibit the final reaction.

Further components may be included in the composition as would be understood in the PCR art. These might include sequences used as internal controls as well as primers for amplifying these sequences and signalling systems such as those outlined above for detecting amplification of the internal control sequences.

Compositions of the invention are suitably prepared by mixing together the required components as described above to form a composition, and adding water, preferably sterile water which been treated with diethyl pyrocarbonate (DEPC) to the composition to allow for mixing, for example by adding at least equivalent volume and preferably from 1-1.5 times the volume of the final reaction composition. In particular, the volume of the cake mixture formed at this stage is more dilute than that which it is intended to be formed for use in the chemical or biochemical reaction itself. In particular sufficient water is added to ensure that the volume of the composition or of each aliquot containing sufficient material for conducting a chemical or biochemical reaction such as PCR, is from 1.5-5 times and suitably about twice the recommended volume of the final reaction composition, intended to carry out the PCR. The thus formed mixture is, if necessary dispensed into suitable aliquots each of which contains sufficient material for a PCR in an individual reaction pot, and then subjected to a freeze drying process. If freeze drying does not take place immediately, the final mixture is suitably stored at low temperatures, for example on ice, or in a freezer if the delay is prolonged beyond about 0.5 hours, until freeze drying takes place.

The freeze-drying protocol used will depend to some extent upon the particular composition being dried and will be determined in each case using routine procedures. Typically, the composition will be subject to a freezing step in which it is cooled to a low temperature for example from about -20°C to -60°C and generally at about -40°C at a pressure of from 300-400torr, and held at this temperature for a sufficient period of time to ensure that complete freezing occurs.

The pressure is then reduced to an appropriate level depending upon the particular freeze-dryer used. Some may operate a pressures as low as 6Mtorr but for current purposes, pressures of from 10 to 100mTorr may be suitable to allow the water to sublimate. Suitably then the composition is brought back to room temperature and pressure. This may be done by gradually bringing the composition back up to room temperature under reduced pressure, before the vacuum is released to minimise condensation effects.

Optionally, the vacuum is released in the presence of an inert atmosphere such as nitrogen, so that the product is maintained in an inert environment. This supports longevity and also prevents moisture ingress.

Freeze-dried product obtained in this way, it is suitably packaged immediately for example in foil wrappers, to minimise the contamination risk. If the composition is contained within containers such as reagent pots, these are suitably sealed before the vacuum is released. Foil wrapper includes packaging made from aluminium foil, aluminium polymer laminated foil or extrusions. It also includes materials such as mylar®, which being strong makes ideal packaging for long term storage. The wrapper provides a secondary barrier to reduce gaseous exchange and moisture ingress.

Care needs to be taken to ensure that all reagents utilised in the composition do not contain materials or contaminants that could inhibit or prevent freeze drying in the levels in which they are found. Thus for example, it may be necessary to remove substances such as glycerol which are sometimes included in commercially available enzymes such as polymerases, reverse transcriptase polymerases and RNase inhibitors, and or to reduce the levels of substances such as dimethyl sulphoxide (DMSO) which may be found in intercalating dyes which may be used as DNA duplex binding agents. This may be achieved through standard methods, for example, as dialysis, ultra separation and exclusion chromatography as is well understood in the art.

Compositions as described above have been found to be stable for extended periods of time, including up to 3 months, and further up to 9 months, at the end of which, no activity loss at all was seen.

Methods for forming compositions described above form a further aspect of the invention. In a particular embodiment, the invention provides a method for preparing a freeze dried composition, which comprises mixing together at least items (i) to (ii) above and freeze drying the resultant mixture.

In use the compositions of the invention are hydrated using conventional methods, for example using a rehydration buffer and then subject to the appropriate chemical or biochemical reaction. Generally, the composition will be mixed with a chemical or biochemical sample before the reaction is conducted. For example, in the case of a polymerase chain reaction, the reaction mixture is combined with a sample which contains or is suspected of containing a target nucleic acid, and optionally also a rehydration buffer and the final mixture subjected to PCR conditions. Where signalling reagents are included, the signal, for example fluorescence from the fluorescent reagent is monitored before, during or after the process as required. In particular, the signal is monitored in real-time as required, so as to allow the progress of the reaction to be monitored and the amount of target in the sample quantified, as is understood in the art. Such methods form a further aspect of the invention.

The invention will now be particular described by way of example with reference to the accompanying Figures in which:

Figure 1 is a graphical comparison of the average Ct values (+/- sd) of freeze dried reagents stored at room temperature and 30°C over a 21 day period; and
Figure 2 is a series of graphs showing the Ct values (+/- sd) and fluorescence values of freeze dried reagents stored at 30°C over a 9 month period.

Example 1

Study of PCR inhibition assay for detecting *Bacillus subtilis var. globigii* (BG) DNA and spores in the presence of Trehalose and Raffinose

A PCR mastermix was prepared by combining multiples of the following reagents for 20µl or 25µl reactions.

Table 1

| Reagent | Conc. | Vol. (µl per 20µl final reaction volume) | Vol. (µl per 25µl final reaction volume) |
|---|---|---|---|
| Tris pH 8.8 | 500mM | 2.0 | 2.5 |
| BSA | 20mg/ml | 0.25 | 0.3 |
| MgCl$_2$ | 100mM | 2.0 | 2.5 |
| Forward primer | 10µM | 2.0 | 2.5 |
| Reverse primer | 10µM | 2.0 | 2.5 |
| Labelled Taqman ® probe | 2µM | 2.0 | 2.5 |
| β-Actin T Probe (JOE) | 2µM | 2.0 | 2.5 |
| *Taq* antibody | 5U/µl filtered | 0.32 | 0.4 |
| *Taq* polymerase | 5U/µl filtered | 0.16 | 0.2 |
| Water | DNAse/RNAse free | Variable | Variable |
| Purified Template BG DNA | 2x10$^5$ to 2x10$^2$ copies per reaction | 2.0 | 2.5 |

To samples of this mastermix, additives, either 50% (m/v) trehalose (2µl for 20µl or 2.5µl for 25µl reaction mixtures) or 25% (m/v) raffinose (4µl for 20µl or 5µl for 25µl reactions) was added to produce 5% (m/v) trehalose or 5% (m/v)raffinose compositions. Control reactions were carried out in the absence of either trehalose or raffinose. A negative control was prepared using water instead of template DNA.

These non-lyophilised formulations were then transferred to Lightcyler ™ capillaries and the capillaries capped. They were briefly centrifuged and loaded into the rotor for the LightCycler 1.0 (Roche) and subjected to a PCR reaction using the following protocol:

| Phase | | | Segment | Target Temp | Hold time | Trans -ition rate | Acqusition | | |
|---|---|---|---|---|---|---|---|---|---|
| No. | Type | No. cycles | No. | °C | s | °C/s | Type | Channels | Gains |
| 1 | Denature | 1 | 1 | 95 | 60 | 10 | | | |
| 2 | Amplify | 50 | 1 | 95 | 5 | 10 | | | |
| | | | 2 | 55 | 30 | 10 | single | ALL | 1.5.15 |

The signals from the Taqman ® probe were read with a single acquisition through each cycle over a range of serial dilutions. The average Ct, fluorescence and signal/noise are compared in Table 1A.

Table 1A

|  | 2 x 10⁵ copies | | | 2 x 10⁴ copies | | |
|---|---|---|---|---|---|---|
|  | Av. Ct | Av. Fluor | Av. signal/ noise | Av. Ct | Av. Fluor | Av. signal/ noise |
| No additive | 29.995 | 24 | 116.05 | 32.835 | 21 | 103.91 |
| 5% Trehalose | 30.525 | 23 | 112.85 | 33.46 | 20 | 51.72 |
| 5% Raffinose | 30.28 | 23 | 108.14 | 32.995 | 20 | 128.44 |

|  | 2 x 10³ copies | | | 2 x 10² copies | | |
|---|---|---|---|---|---|---|
|  | Av. Ct | Av. Fluor | Av. signal/ noise | Av. Ct | Av. Fluor | Av. signal/ noise |
| No additive | 35.25 | 14 | 51.01 | 36.095 | 2 | 6.69 |
| 5% Trehalose | 35.73 | 11 | 51.35 | 36.06 | 3 | 13.12 |
| 5% Raffinose | 35.14 | 12 | 48.67 | 35.7 | 3 | 17.5 |

The experiment was repeated with a different set of BG primers and probes and the results are summarised in Table 2.

Table 2

| | 2 x $10^5$ copies | | | 2 x $10^4$ copies | | |
|---|---|---|---|---|---|---|
| | Av. Ct | Av. Fluor | Av. signal/ noise | Av. Ct | Av. Fluor | Av. signal/ noise |
| No additive | 24.16 | 8.5 | 23.42 | 27.755 | 8.5 | 22.94 |
| 5% Trehalose | 24.495 | 8 | 18.05 | 27.855 | 7.5 | 20.02 |
| 5% Raffinose | 24.21 | 7.5 | 32.12 | 27.56 | 7.5 | 23.42 |

| | 2 x $10^3$ copies | | | 2 x $10^2$ copies | | |
|---|---|---|---|---|---|---|
| | Av. Ct | Av. Fluor | Av. signal/ noise | Av. Ct | Av. Fluor | Av. signal/ noise |
| No additive | 30.985 | 8 | 18.27 | 34.47 | 6.5 | 14.42 |
| 5% Trehalose | 30.895 | 6.5 | 16.3 | 34.035 | 5.5 | 12.97 |
| 5% Raffinose | 30.92 | 7 | 28.94 | 34.36 | 5 | 19.99 |

The results showed that neither assay were adversely affected by the addition of 5% (m/v) trehalose or 5% (m/v) raffinose with no difference in Ct value, average fluorescence or signal/noise observed at any concentration of BG DNA.

Example 2

Study of PCR inhibition assay for detecting *Bacillus subtilis var. globigii* (BG) DNA and spores in the presence of Trehalose and Raffinose following freeze drying

As neither raffinose or trehalose inhibited either of the assays in a non-lyophilised formulations /reaction mix, these excipients were tested as the freeze dried formulation with or without the presence of 1% (m/v) PEG (final). Reaction mixtures as described in Example 1 but with or without the addition of 1%(m/v) PEG were prepared and freeze dried using a VirTis Advantage freeze drier, which was set to carry out the program summarised in Table 3.

Table 3

| Step | Temp°C | Time (min) | Pressure (Torr) | Ramp/Hold |
|---|---|---|---|---|
| **Thermal Treatment** | | | | |
| 1 | +10 | 15 | 3-400 | H |
| 2 | -40 | 60 | 3-400 | R |
| 3 | -40 | 180 | 3-400 | H |
| Freeze, condenser, vac | -40 freeze & condense | 0 | 100mTorr | |
| **Primary Drying** | | | | |
| 1 | -40 | 45 | 100mTorr | H |
| 2 | +5 | 55 | 100mTorr | R |

(continued)

| Primary Drying | | | | |
|---|---|---|---|---|
| 3 | +5 | 30 | 100mTorr | H |
| 4 | +20 | 25 | 100mTorr | R |
| 5 | +20 | 240 | 100mTorr | H |
| 6 | +5 | 25 | 100mTorr | R |
| 7 | +5 | 500 | 100mTorr | H |
| 8 | +20 | 25 | 100mTorr | R |
| 9 | +20 | 15 | 100mTorr | H |
| 10 | +10 | 20 | 100mTorr | R |
| 11 | +10 | 1000 | 100mTorr | H |
| Secondary Drying | +27 set point | | | |
| Post Heat Settings | +10 | 1000 | 100mTorr | |

The resultant freeze dried mixes (cakes) were immediately resuspended in buffer and subjected to a PCR protocol as outlined in Example 1. All cakes were prepared as 50µl solutions (2x) and resuspended to 25µl.

The showing a comparison of average Ct, fluorescence and signal/noise reaction for the two Taqman ® BG assays are set out in Table 4 and 5 respectively.

Table 4

| | $2 \times 10^5$ copies BG DNA | | | $2 \times 10^4$ copies BG DNA | | |
|---|---|---|---|---|---|---|
| | Av. Ct | Av. Fluor | Av.signal/ noise | Av. Ct | Av. Fluor | Av.signal/ noise |
| Non-lyophilised | 31.355 | 19 | 81.42 | 35.085 | 20 | 93.19 |
| Trehalose | 31.16 | 17.5 | 131.92 | 34.51 | 16 | 81.03 |
| Trehalose + PEG | 31.91 | 13.5 | 198 | 36.05 | 10.5 | 49.62 |
| Raffinose | 32.61 | 9 | 60.61 | 35.58 | 11 | 68.64 |
| Raffinose + PEG | 30.01 | 14 | 79.19 | 33.045 | 13 | 116.11 |

Table 5

| | $2 \times 10^5$ copies BG DNA | | | $2 \times 10^4$ copies BG DNA | | |
|---|---|---|---|---|---|---|
| | Av. Ct | Av. Fluor | Av.signal/ noise | Av. Ct | Av. Fluor | Av.signal/ noise |
| Non-lyophilised | 26.245 | 6.25 | 27.3845 | 29.635 | 6.5 | 27.636 |
| Trehalose | 27.025 | 3.75 | 14.36 | 30.46 | 4.375 | 11.93 |
| Trehalose + PEG | 26.175 | 3.33 | 63.29 | 28.87 | 3.25 | 34.93 |
| Raffinose | 24.88 | 6.25 | 9.22 | 28.64 | 4.5 | 12.05 |
| Raffinose + PEG | 25.135 | 5.75 | 36.46 | 29.455 | 4.5 | 32.65 |

Positive PCR curves were obtained for both assays using all four formulations, with no obvious differences in Ct values for any of the mixes.

In the first assay, the formulations containing trehalose in the absence of PEG and raffinose including 1% (m/v) PEG had similar signal/noise values to the non-lyophilised (wet) formulation controls for both concentrations of BG DNA (Table 4 above). In the second assay, formulations containing raffinose either alone or with 1% (m/v) PEG kept the signal/noise values closest to those observed in non-lyophilised formulations mixes, indicating that these were preferred mixes.

Example 3

Evaluation of Raffinose as a glassing agent and stabiliser in a composition for detecting BG in a Dual Hybridisation assay.

The procedure of Example 1 was broadly followed, except that a dual hybridisation probe pair were used instead of the single Taqman ® labelled probe, and using the reagents listed in Table 6. In this case, the probes were designed to hybridise to the amplified BG DNA so that the FAM and Cy5 labels were brought into close proximity to each other.

Table 6

| Reagent | Conc. | Vol. ($\mu$l per 25$\mu$l cake volume) | Final concentration in reaction (25 $\mu$l) |
|---|---|---|---|
| Tris pH 8.8 | 500mM | 2.5 | 50mM |
| BSA | 20mg/ml | 0.31 | 0.25mg/ml |
| $MgCl_2$ | 100mM | 0.75 | 3mM |
| dUTP mix | 2mM | 2.5 | 0.2mM |
| Forward primer | 10$\mu$M | 2.5 | 1$\mu$M |
| Reverse primer | 10$\mu$M | 2.5 | 1$\mu$M |
| FAM labelled Donor probe | 2$\mu$M | 2.5 | 0.2$\mu$M |
| Cy5 labelled acceptor probe | 2$\mu$M | 2.5 | 0.2$\mu$M |
| Trehalose or raffinose | 50%(m/v) 25%(m/v) | See table 7 | See table 7 |
| With or without PEG 20,000 | 10%(m/v) | 7.5 | 1%(m/v) |
| RI Out ribonuclease inhibitor | 5U/$\mu$l filtered | 0.3 | 0.04U/$\mu$l |
| *Taq* antibody | 5U/$\mu$l filtered | 0.4 | 0.08U/$\mu$l |
| *Taq* polymerase | 5U/$\mu$l filtered | 0.25 | 0.04U/$\mu$l |
| Water | DEPC treated | 27.9 | |

As before, once these reagents had been combined in a reaction tube, they were placed inside a freeze dryer (Virtis Advantage), which was previously chilled to +5°C and then set to carry out the program summarised in Table 3 above.

Five replicates of each mix was prepared. Analysis of the appearance of the freeze dried cakes was made and the results summarised in Table 7 below.

These were then further compared by reconstitution of the dried mix and addition of BG DNA. Each mix was then subjected to a PCR in a LightCycler™ using the following protocol:

| Phase | | | Segment | Target Temp | Hold time | Trans -ition rate | Acqusition | | |
|---|---|---|---|---|---|---|---|---|---|
| No. | Type | No. cycles | No. | °C | s | °C/s | Type | Channels | Gains |
| 1 | Denature | 1 | 1 | 95 | 60 | 10 | | | |
| 2 | Amplify | 50 | 1 | 95 | 5 | 10 | | | |
| | | | 1 | 55 | 20 | 10 | | | |
| | | | 2 | 74 | 5 | 10 | single | ALL | 1.5.15 |

The results for the mean Ct values and the mean signal for the 5 replicates is summarised in Table 7.

Table 7: Observations of attributes of freeze dried cakes prepared with different concentrations of trehalose and raffinose.

| | Neat BG DNA | | Observation of freeze dried cake |
|---|---|---|---|
| | Mean Ct | Mean Signal | |
| 1% trehalose | 23.73 | 26.141 | wet looking, hole in centre shrivelled and disappeared on air exposure |
| 2.5% trehalose | 23.92 | 29.886 | less wet looking than 1%, still pit in centre, shank very quickly on air exposure |
| 5% trehalose | 24.38 | 19.877 | looked similar to standard FD cake shrunk but still there 2hrs post freeze drying |
| 10% trehalose | 24.46 | 17.056 | looked similar to standard FD cake shrunk but still there 2hrs post freeze drying |
| | Mean Ct | Mean Signal | |
| BGDH standard | 24.61 | 14.568 | looked dry shrunk least of trehalose batch on air exposure |
| 1% raffinose | 23.45 | 22.146 | wet looking, shrunk similarly to 1% trehalose on air exposure |
| 2.5% raffinose | 23.8 | 23.283 | wet looking, shrunk similar to 1% trehalose on air exposure |
| 5% raffinose | 24.2 | 25.269 | looked dry similar to trehalose 5%, on air exposure cake had diasppeared by 2hrs |
| 10% raffinose | 24.3 | 16.131 | hardly shrunk after 2hrs air exposure |
| Raffinose + 1% PEG | 23.3 | 16.856 | hardly shrunk after 2 hrs air exposure |

The freeze-dried cakes appeared drier as the concentration of the trehalose or raffinose increased with no obvious differences between the two. The Ct values were similar both between raffinose and trehalose and within the different concentrations of the two agents. Generally, the results for raffinose were similar to those of trehalose, indicating that it can be replaced in freeze-dried formulations without adverse effects. However, raffinose has the added property of being an *in situ* dessicant, and so would be expected to improve the stability of the compositions.

Example 6

Effect of Threonine on the compositions

A Taqman ® mixture was formulated with the components listed in Table 8:

**Table 8**

| Reagent | Conc. | Vol per Cake (µl) |
|---|---|---|
| Tris pH 8.8 | 500mM | 2.5 |
| BSA | 20mg/ml | 0.3 |
| $MgCl_2$ | 100mM | 0.75 |
| dUTP mix | 2mM | 2.5 |
| Forward primer | 10µM | 2.5 |
| Reverse primer | 10µM | 2.5 |
| Raffinose | 25%(m/v) | 5.0 |

(continued)

| Reagent | Conc. | Vol per Cake ($\mu$l) |
|---|---|---|
| L-threonine* | 2.5mM | 0.16 |
| PEG | 10%(m/v) | 2.5 |
| *Taq* antibody | 5U/$\mu$l filtered | 0.4 |
| *Taq* polymerase | 5U/$\mu$l filtered | 0.2 |
| Taqman ® probe | 2$\mu$M | 2.5 |
| Internal control probe | 2$\mu$M | 2.5 |
| Water | DEPC treated | to 50$\mu$l |

A similar mixture was created without the L-threonine. Both mixtures were then freeze-dried as described in Example 2. The double foiled pots containing the freeze dried reagents were placed in plastic Petri dishes at room temperature and at 30°C. The room temperature pots were stored in a non temperature controlled laboratory in a cupboard. Each of the freeze dried reagent formulations were tested on day 0 and then on days, 1, 6, 9, 14, 21 and 28, by running a PCR using the following protocol with final reaction volume of 25$\mu$l.

| Phase | | | Segment | Target Temp | Hold time | Transition rate | Acqusition | | |
|---|---|---|---|---|---|---|---|---|---|
| No. | Type | No. cycles | No. | °C | s | °C/s | Type | Channels | Gains |
| 1 | Denature | 1 | 1 | 95 | 60 | 10 | | | |
| 2 | Amplify | 50 | 1 | 95 | 5 | 10 | | | |
| | | | 2 | 60 | 30 | 10 | single | ALL | 1.5.15 |

The results were compared to that of a freshly prepared composition. The Ct, signal and signal to noise values were determined using the LightCycler® 1.0 data. A signal to noise value was generated using the following equation:-

$$\text{Signal to noise value} = \frac{(\text{signal} - \text{noise})}{\text{noise}}$$

where signal = the last value recorded (at cycle 50)
noise = the difference between the highest and lowest values recorded over the first 10 cycles

The results for the compositions stored at room temperature are shown in Table 9 and those stored at 30°C in Table 10.

Table 9

| Storage | | Day 0 | | | Day 1 | | |
|---|---|---|---|---|---|---|---|
| | | Av. Ct | Av. Fluor | Av.signal/ noise | Av. Ct | Av. Fluor | Av. Signal/ noise |
| Non-lyophilised | | 30.73 | 17.15 | 104.59 | 30.06 | 20.265 | 69.53 |
| FD reagent -L Threonine | FD lab RT | 28.98 | 11.57 | 121.85 | 28.61 | 13.4278 | 108.09 |
| FD reagent +L Threonine | FD lab RT | 29.84 | 11.66 | 90.22 | 28.95 | 15.466 | 100.91 |

| Storage | | Day 6 | | | Day 9 | | |
|---|---|---|---|---|---|---|---|
| | | Av. Ct | Av. Fluor | Av.signal/ noise | Av. Ct | Av. Fluor | Av.signal/ noise |
| Non-lyophilised | | 29.35 | 20.18 | 87.68 | 30 | 18.957 | 74.97 |
| FD reagent -L Threonine | FD lab RT | 28.69 | 12.933 | 155.29 | 28.92 | 12.1824 | 167.29 |
| FD reagent +L Threonine | FD lab RT | 29.45 | 11.816 | 108.4 | 30.1 | 11.341 | 137.2 |

| Storage | | Day 14 | | | Day 21 | | |
|---|---|---|---|---|---|---|---|
| | | Av. Ct | Av. Fluor | Av.Signal /Noise | Av. Ct | Av. Fluor | Av. Signal /Noise |
| Non-lyophilised | | 29.93 | 20.089 | 70.92 | 29.79 | 20.6398 | 57.23 |
| FD reagent -L Threonine | FD lab RT | 29.05 | 11.967 | 105.6 | 28.64 | 13.3 | 146.68 |
| FD reagent +L Threonine | FD lab RT | 29.18 | 14.3 | 87.54 | 29.32 | 14.67 | 121.1 |

| storage | | Day 28 | | |
|---|---|---|---|---|
| | | Av. Ct | Av. Fluor | Av. Signal/Noise |
| Non-lyophilised | | 29.45 | 18.77 | 75.02 |
| FD reagent -L Threonine | FD lab RT | 28.56 | 13.5 | 266.02 |
| FD reagent +L Threonine | FD lab RT | 29.29 | 12.428 | 200.78 |

**Table 10.**

| Storage | | Day 0 | | | Day 6 | | |
|---|---|---|---|---|---|---|---|
| | | Av. Ct | Av. Fluor | Av. Signal/ noise | Av. Ct | Av. Fluor | Av. Signal/ Noise |
| Non-lyophilised | | 30.73 | 17.15 | 104.59 | 29.35 | 20.18 | 87.68 |
| FD reagent – L threonine | FD lab RT | 28.98 | 11.57 | 121.85 | 28.69 | 12.93287 | 155.29 |
| FD reagent +L Threonine | FD lab RT | 29.84 | 11.66 | 90.22 | 29.45 | 11.816 | 108.4 |
| FD reagent – L Threonine | $30^o$ C | 28.98 | 11.57 | 121.85 | 30.14 | 8.24 | 62.22 |
| FD reagent +L Threonine | $30^0$ C | 29.84 | 11.66 | 90.22 | 30.28 | 9.69 | 90.11 |

|  | Storage | Day 9 | | | Day 21 | | |
|---|---|---|---|---|---|---|---|
|  |  | Av. Ct | Av. Fluor | Av. Signal/ noise | Av. Ct | Av. Fluor | Av. Signal/ Noise |
| Non-lyophilised |  | 30 | 18.957 | 74.97 | 29.79 | 20.6398 | 57.23 |
| FD reagent – L Threonine | FD lab RT | 28.92 | 12.182 | 167.29 | 28.64 | 13.3 | 146.68 |
| FD reagent +L Threonine | FD lab RT | 30.1 | 11.341 | 137.2 | 29.32 | 14.67 | 121.1 |
| FD reagent – L Threonine | 30° C | 30.22 | 8.046 | 63.47 | 29.01 | 11.088 | 122.4 |
| FD reagent +L Threonine | 30° C | 30.19 | 10.8 | 78.62 | 29.26 | 14.893 | 138.17 |

The results suggest that both formulations were stable at room temperature over the period of the test. When stored at 30°C, the results showed that the compositions were stable for 21 days.

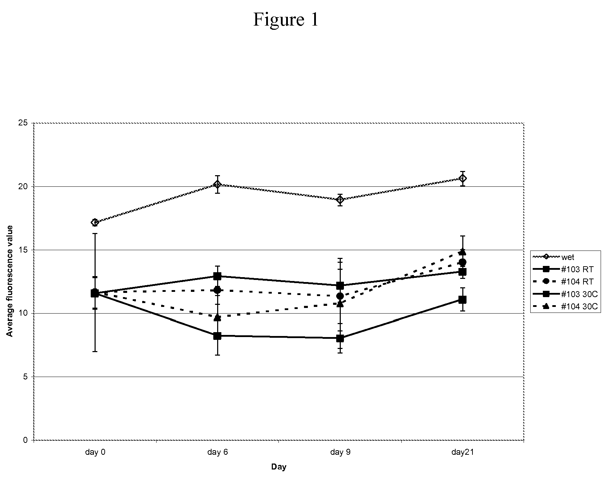

However, the formulation containing L-threonine had better signalling at all time points when stored at 30°C (see Figure 1). The signal from the L-threonine containing composition was clearly less inhibited that that from the similar composition from which L-threonine was omitted. Hence, it appears that the use of L-threonine will not only assist the stability as a result of the anti-oxidant/anti maillard properties, but also that it will reduce the loss of fluorescent signals.

Example 7

Comparison of Raffinose and Trehalose as a glassing agent and stabiliser in a composition for an RT-PCR assay.

The compositions set out in Table 11 were prepared.

Table 11

| Reagent | Concentration | Trehalose Cake Formulation (50 μl Total) (Reaction volume 25μl) | | Raffinose Cake Formulation (50 μl Total) (Reaction volume 25μl) |
| --- | --- | --- | --- | --- |
| | | Mix A (μl) | Mix B(μl) | Mix A(μl) |
| RT Buffer (Genesys Ltd)* | 5x | 5 | | 5 |
| MnCl$_2$ | 50 mM | 1 | | 1 |
| Forward Primer A11 | 10μM | | 0.3 | 0.3 |
| Reverse Primer A14 | 10μM | | 0.3 | 0.3 |
| Trehalose | 50 % (m/v) | 2.5 | 2.5 | |
| Raffinose | 25 % (m/v) | | | 5 |
| Polyethylene Glycol | 10 % (m/v) | 5 | 5 | 2.5 |
| RNase Inhibitor | 5 units /μl | 0.2 | 0.2 | 0.2 |
| L-Threonine | 400 mM | 0.6 | 0.6 | 0.6 |
| Tsec | 5 units/ μl | 0.3 | | 0.3 |
| Probe BVDV1/2 | 2μM | | 3 | 3 |
| RNase/DNase Free Water | N/A | 10.4 | 13.1 | 31.8 |

* RT Buffer formulation is 600 mM Potassium Acetate (120mM final), 200mM Bicine (40mM final), Bovine Serum Albumin 2.5 mmg/ml (500 ng/ μl final), and 1 mM dNTP (20μM final).

They were then freeze dried as described in Example 2 above. The trehalose cake formulation was prepared by mixing together mix A & B prior to freeze drying. In both cases (trehalose and raffinose) the cake formulation at this stage was twice (50 μl) the volume of the actual reaction volume (25 μl).

Post freeze drying the following observations were made of the trehalose and raffinose product. The trehalose cake had a clear "toffee" consistency which had not formed a cake. Dissolution was difficult due to the nature of the resultant cake. The raffinose cake had formed although it appeared to have collapsed a little. However, it was easy to resuspend and thus preferable to handle.

The compositions of Table 11 were then reconstituted by addition of water and DNA at the required concentration to a total volume of 25μl water.

Thes compositions, as well as compositions that were prepared fresh and were not subject to freeze drying, were then subjected to an RT-PCR. Samples were amplified on a Roche LightCycler 1.0 instrument using the following thermal and optical protocol.

| Phase | | | Segment | Target Temp | Hold time | Transition rate | Acqusition | | |
|---|---|---|---|---|---|---|---|---|---|
| No. | Type | No. cycles | No. | °C | s | °C/s | Type | Channels | Gains |
| 1 | Denature | 1 | 1 | 60 | 120 | 10 | | | |
| 2 | Amplify | 50 | 1 | 95 | 10 | 10 | | | |
| | | | 2 | 60 | 30 | 10 | single | ALL | 1.5.15 |

The results are summarised in the following tables.

| Ct Values | | | | | | |
|---|---|---|---|---|---|---|
| | Trehalose | | | Raffinose | | |
| copy number BVDV RNA | $6 \times 10^6$ | $6 \times 10^5$ | $6 \times 10^4$ | $6 \times 10^6$ | $6 \times 10^5$ | $6 \times 10^4$ |
| non-lyophilised formulation no excipients | 31.47 | 34.02 | 36.24 | 30.7 | 33.74 | 35.98 |
| non-lyophilised formulation + excipients | 30.27 | 33.28 | 35.32 | 30.18 | 33.4 | 35.74 |
| Freeze Dried | 35.97 | 38.98 | Neg | 33.54 | 35 | Neg |
| **Fluorescence Values** | | | | | | |
| copy number BVDV RNA | Trehalose | | | Raffinose | | |
| | $6 \times 10^6$ | $6 \times 10^5$ | $6 \times 10^4$ | $6 \times 10^6$ | $6 \times 10^5$ | $6 \times 10^4$ |
| non-lyophilised formulation no excipients | 26 | 20 | 4.1 | 47 | 33 | 9 |
| non-lyophilised formulation + excipients | 19 | 13 | 1.7 | 52 | 25 | 5.5 |
| Freeze Dried | 7 | 4.5 | Neg | 18 | 10 | Neg |

In these tables "non-lyophilised formulation" refers to the compositions that had not been subject to freeze drying. The 'excipients' refers to the inclusion of either trehalose or Raffinose according to the experiment in each column

The raffinose compositions consistently gave results that were more in line with those of the non-lyophilised formulation compositions and therefore are considered to be the preferred formulation.

Example 8

Long term stability of compositions of the invention

A competitive duplex Taqman ® mixture including plasmid DNA as an internal control for the final assay, was formulated with the components listed in Table 12:

**Table 12**

| Reagent | Conc. | Vol (µl) required for cake formulation. Final vol 50µl |
|---|---|---|
| Tris pH 8.8 | 500mM | 2.5 |
| BSA | 20mg/ml | 0.3 |
| $MgCl_2$ | 100mM | 0.75 |
| dUTP mix | 2mM | 2.5 |
| Forward primer | 10µM | 2.5 |
| Reverse primer | 10µM | 2.5 |
| Raffinose | 25%(m/v) | 5.0 |

(continued)

| Reagent | Conc. | Vol (μl) required for cake formulation. Final vol 50μl |
|---|---|---|
| PEG | 10% (m/v) | 2.5 |
| L-threonine | 400mM | 0.15 |
| anti*Taq* antibody | 5U/μl filtered | 0.6 |
| *Taq* polymerase | 5U/μl filtered | 0.3 |
| Taqman ® probe | 2μM | 2.5 |
| Internal control probe | 2μM | 2.5 |
| RNase/Dnase free Water | 22.9 | 22.9 |
| Internal control DNA 3750 copies | 3750 copies/μl | 2.5 |

The mixture was freeze dried as described in Example 2.

The freeze dried reagent formulations were stored at 30°C and the stability tested over a period of 9 months by running a PCR using the following protocol with final reaction volume of 25μl.

| Phase | | | Segment | Target Temp | hold time | Trans -ition rate | Acqusition | | |
|---|---|---|---|---|---|---|---|---|---|
| No. | Type | No. cycles | No. | °C | s | °C/s | Type | Channels | Gains |
| 1 | Hold | 1 | 1 | 95 | 60 | 10 | | | |
| 2 | Amplify | 50 | 1 | 95 | 5 | 10 | | | |
| | | | 2 | 60 | 30 | 10 | single | ALL | 1.5.15 |

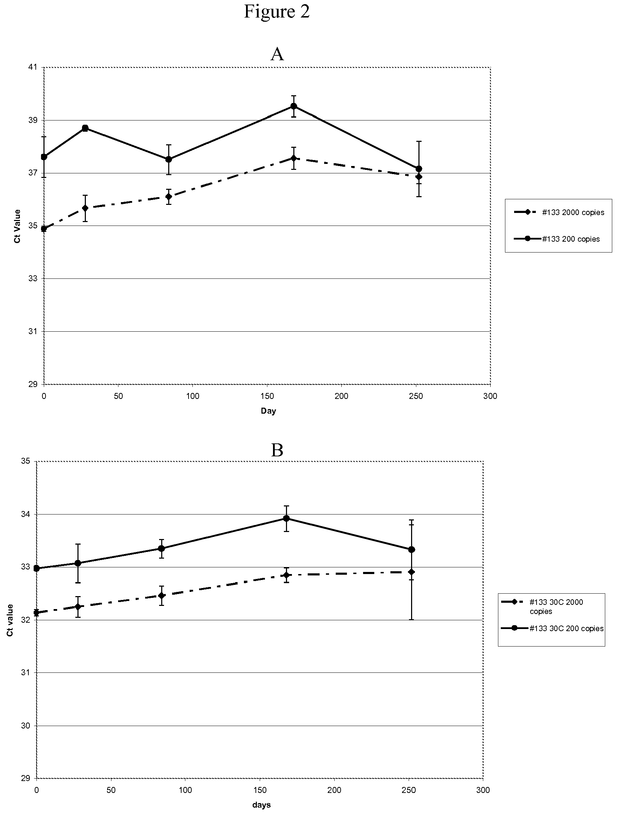

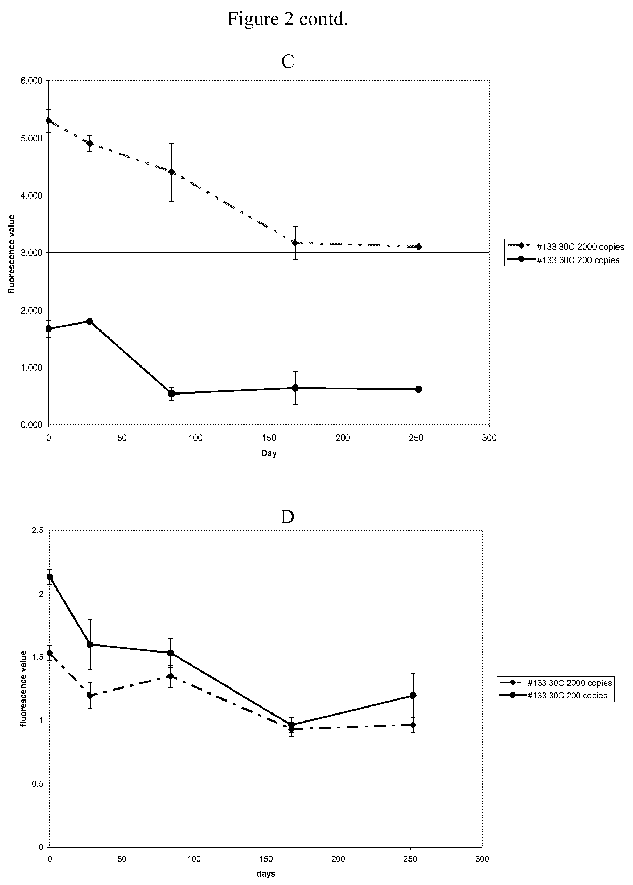

The results are shown in Figure 2. Figures 2A and 2B show the Ct values achieved over a nine month period with storage at 30°C. The data shows results for the amplification using the specific template DNA (added) and internal control DNA (included in the freeze dried cake) respectively. Figures 2C and 2D show the fluorescence values (highest average at plateau) over the nine month period for the template DNA and internal control DNA respectively.

Two concentrations of DNA and approximately 2000 (broken line, diamond) and 200 copies (solid line, circles) were used at each time point and the PCR repeated in triplicate, with the graph showing the mean (+/-) 1 standard deviation at each time point.

Although the Ct values were variable, there was no obvious difference in the Ct values in the lowest concentration of DNA between day 0 and 9 months for either the template DNA or for the IC control DNA. The fluorescence values decreased slightly over time, but did not result in a negative result for either the template or internal control amplification.

**Claims**

1.   A composition for carrying out a polymerase chain reaction (PCR) or a reverse transcriptase polymerase chain reaction (RT-PCR) that may be monitored in real-time, said composition being in a freeze-dried form and comprising (i) a set of reagents comprising at least some of the chemical or biochemical reagents necessary for conducting said PCR or RT-PCR, including a polymerase capable of extending a primer when adhered to a template nucleic acid sequence during the PCR (ii) at least one fluorescent reagent used to monitor the progress of the PCR or RT-PCR in real time and (iii) raffinose;
wherein the raffinose is present in an amount such that in a final composition, reconstituted from said composition in order to carry out a PCR or RT-PCR, it is present in an amount of from 2.5-10%(m/v).

2.   A composition according to claim 1 which further comprises an anti-oxidant or anti-maillard agent.

3.   A composition according to claim 2 wherein the anti-oxidant or anti-maillard agent is threonine.

4.   A composition according to any one of the claims 1 to 3 which further comprises a stabiliser for a glass forming agent.

5. A composition according to claim 4 wherein the stabiliser is selected from polyethylene glycol (PEG), polyvinylpyrrolidine (PVP) or polysaccharide.

6. A composition according to any preceding claim wherein the mixture of (i) includes buffer, primers and nucleotides suitable for conducting a polymerase chain reaction to amplify a DNA sequence.

7. A composition according to any preceding claim wherein the fluorescent reagent is selected from a fluorescent dye or DNA binding agent or a fluorescently labelled oligonucleotide.

8. A composition according to claim 7 wherein the fluorescent reagent is a labelled oligonucleotide that acts as a probe in the assay and carries two labels, one of which is able to act as a donor of energy and one of which is able to act as an acceptor of that energy.

9. A composition according to claim 8 which comprises a pair of labelled probes one of which carries a label with is a fluorescent energy donor and one of which carries a label which is able to accept fluorescent energy from said energy donor, and wherein the probes hybridise in close proximity on a PCR product strand.

10. A method for preparing a composition according to any one of claims 1 to 9, said method comprising mixing together components of the composition together with water and freeze drying the resultant mixture.

11. A method according to claim 10 wherein the concentration of said resultant mixture is less than that of the recommended final composition that is reconstituted from the freeze dried composition.

12. A kit comprising a composition according to any one of claims 1 to 9 and a rehydration buffer.

13. A kit according to claim 12 wherein the composition does not contain salts necessary for carrying out a PCR, and the rehydration buffer includes said salts.

14. A method for conducting a real-time polymerase chain reaction (PCR) or a reverse transcriptase polymerase chain reaction (RT-PCR) which comprises hydrating a composition according to any one of claims 1 to 9 and subjecting this to conditions under which a polymerase chain reaction (PCR) or a reverse transcriptase polymerase chain reaction (RT-PCR) will occur whilst monitoring the progress of the reaction by monitoring fluorescence from the reaction mixture.

**Patentansprüche**

1. Zusammensetzung zur Durchführung einer Polymerase-Kettenreaktion (PCR) oder einer Reverse-Transkriptase-Polymerase-Kettenreaktion (RT-PCR), die in Echtzeit überwacht werden kann, wobei die Zusammensetzung in gefriergetrockneter Form vorliegt und (i) ein Set von Reagenzien, umfassend zumindest einige der zum Durchführen der PCR oder RT-PCR erforderlichen chemischen oder biochemischen Reagenzien, einschließlich einer Polymerase, die imstande ist, einen an eine Matrizen-Nukleinsäuresequenz gebunden Primer während der PCR zu verlängern, (ii) mindestens ein fluoreszierendes Reagenz, das zur Überwachung des Fortschritts der PCR oder RT-PCR in Echtzeit benutzt wird, und (iii) Raffinose umfasst, wobei die Raffinose in einer solchen Menge vorliegt, dass eine aus der Zusammensetzung zur Durchführung einer PCR oder RT-PCR rekonstituierte endgültige Zusammensetzung eine Menge von 2,5 - 10%(m/V) enthält.

2. Zusammensetzung nach Anspruch 1, die ferner ein Antioxidationsmittel oder Anti-Maillard-Mittel umfasst.

3. Zusammensetzung nach Anspruch 2, wobei es sich bei dem Antioxidationsmittel oder Anti-Maillard-Mittel um Threonin handelt.

4. Zusammensetzung nach einem der Ansprüche 1 bis 3, die ferner einen Stabilisator für einen Glasbildner umfasst.

5. Zusammensetzung nach Anspruch 4, wobei der Stabilisator ausgewählt ist aus Polyethylenglykol (PEG), Polyvinylpyrrolidin (PVP) oder Polysaccharid.

6. Zusammensetzung nach einem der vorhergehenden Ansprüche, wobei die Mischung gemäß (i) Puffer, Primer und Nukleotide beinhaltet, die sich dazu eignen, eine Polymerase-Kettenreaktion zum Amplifizieren einer DNA-Sequenz durchzuführen.

7. Zusammensetzung nach einem der vorhergehenden Ansprüche, wobei das fluoreszierende Reagenz ausgewählt ist aus einem fluoreszenten Farbstoff oder einem DNA-Bindemittel oder einem fluoreszenzmarkierten Oligonukleotid.

8. Zusammensetzung nach Anspruch 7, wobei es sich bei dem fluoreszierenden Reagenz um ein markiertes Oligonukleotid handelt, das als Sonde in dem Assay fungiert und zwei Markierungen trägt, von denen eine in der Lage ist, als Energiedonor zu fungieren, und eine in der Lage ist, als Akzeptor dieser Energie zu fungieren.

9. Zusammensetzung nach Anspruch 8, die ein Paar von markierten Sonden umfasst, von denen eine eine Markierung trägt, die ein Fluoreszenzenergiedonor ist, und von denen eine eine Markierung trägt, die in der Lage ist, von dem Energiedonor Fluoreszenzenergie aufzunehmen, und wobei die Sonden in unmittelbarer Nähe an einem PCR-Produktstrang hybridisieren.

10. Verfahren zur Herstellung einer Zusammensetzung nach einem der Ansprüche 1 bis 9, umfassend Vermischen von Komponenten der Zusammensetzung zusammen mit Wasser und Gefriertrocknen der resultierenden Mischung.

11. Verfahren nach Anspruch 10, wobei die Konzentration der resultierenden Mischung kleiner ist als die der empfohlenen endgültigen Zusammensetzung, die aus der gefriergetrockneten Zusammensetzung rekonstituiert wird.

12. Kit, umfassend eine Zusammensetzung nach einem der Ansprüche 1 bis 9 und einen Rehydratationspuffer.

13. Kit nach Anspruch 12, wobei die Zusammensetzung keine Salze enthält, die zur Durchführung einer PCR notwendig sind, und der Rehydratationspuffer diese Salze beinhaltet.

14. Verfahren zur Durchführung einer Echtzeit-Polymerase-Kettenreaktion (PCR) oder einer Reverse-Transkriptase-Polymerase-Kettenreaktion (RT-PCR), umfassend Hydratisieren einer Zusammensetzung nach einem der Ansprüche 1 bis 9 und Aussetzen derselben Bedingungen, unter welchen eine Polymerase-Kettenreaktion (PCR) oder eine Reverse-Transkriptase-Polymerase-Kettenreaktion (RT-PCR) erfolgt, während der Fortschritt der Reaktion durch Überwachen von Fluoreszenz aus der Reaktionsmischung verfolgt wird.

**Revendications**

1. Composition permettant de mettre en oeuvre une réaction en chaîne par polymérase (PCR) ou une réaction en chaîne par polymérase après transcription inverse (RT-PCR) qui peut être suivie en temps réel, ladite composition étant sous une forme lyophilisée et comprenant (i) un ensemble de réactifs comprenant au moins certains des réactifs chimiques ou biochimiques nécessaires pour mettre en oeuvre ladite PCR ou RT-PCR, notamment une polymérase capable d'allonger une amorce quand elle adhère à une séquence d'acide nucléique de matrice pendant la PCR, (ii) au moins un réactif fluorescent utilisé pour suivre l'évolution de la PCR ou de la RT-PCR en temps réel et (iii) du raffinose ; dans laquelle le raffinose est présent en une quantité telle que dans une composition finale, reconstituée à partir de ladite composition afin de mettre en oeuvre une PCR ou une RT-PCR, il est présent en une quantité de 2,5 % à 10 % (m/v).

2. Composition selon la revendication 1, qui comprend en outre un agent antioxydant ou anti-maillard.

3. Composition selon la revendication 2, dans laquelle l'agent antioxydant ou anti-maillard est la thréonine.

4. Composition selon l'une quelconque des revendications 1 à 3, qui comprend en outre un agent de stabilisation pour un agent vitrifiant.

5. Composition selon la revendication 4, dans laquelle l'agent de stabilisation est choisi parmi le polyéthylène glycol (PEG), la polyvinylpyrrolidine (PVP) ou un polysaccharide.

6. Composition selon l'une quelconque des revendications précédentes, dans laquelle le mélange de (i) comprend un tampon, des amorces et des nucléotides appropriés pour mettre en oeuvre une réaction en chaîne par polymérase afin d'amplifier une séquence d'ADN.

**7.** Composition selon l'une quelconque des revendications précédentes, dans laquelle le réactif fluorescent est choisi parmi un colorant fluorescent ou un agent de liaison à l'ADN ou un oligonucléotide à marquage fluorescent.

**8.** Composition selon la revendication 7, dans laquelle le réactif fluorescent est un oligonucléotide marqué qui joue le rôle de sonde dans l'essai et porte deux marqueurs parmi lesquels un est capable de jouer le rôle de donneur d'énergie et un est capable de jouer le rôle d'accepteur de cette énergie.

**9.** Composition selon la revendication 8, qui comprend une paire de sondes marquées parmi lesquelles une porte un marqueur qui est un donneur d'énergie de fluorescence et une porte un marqueur qui est capable d'accepter l'énergie de fluorescence provenant dudit donneur d'énergie, et dans laquelle les sondes s'hybrident à proximité immédiate à un brin de produit PCR.

**10.** Méthode de préparation d'une composition selon l'une quelconque des revendications 1 à 9, ladite méthode comprenant le mélange ensemble des composants de la composition avec de l'eau et la lyophilisation du mélange résultant.

**11.** Méthode selon la revendication 10, dans laquelle la concentration dudit mélange résultant est inférieure à celle de la composition finale recommandée qui est reconstituée à partir de la composition lyophilisée.

**12.** Kit comprenant une composition selon l'une quelconque des revendications 1 à 9 et un tampon de réhydratation.

**13.** Kit selon la revendication 12, dans lequel la composition ne contient pas les sels nécessaires pour mettre en oeuvre une PCR, et le tampon de réhydratation comprend lesdits sels.

**14.** Méthode permettant de mettre en oeuvre une réaction en chaîne par polymérase en temps réel (PCR) ou une réaction chaîne par polymérase après transcription inverse (RT-PCR), qui comprend l'hydratation d'une composition selon l'une quelconque des revendications 1 à 9 et la soumission de celle-ci à des conditions dans lesquelles une réaction en chaîne par polymérase (PCR) ou une réaction en chaîne par polymérase après transcription inverse (RT-PCR) a lieu, tout en suivant l'évolution de la réaction en surveillant la fluorescence provenant du mélange réactionnel.

Figure 1

# Figure 2

## A

## B

Figure 2 contd.

C

D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5250429 A
- US 5763157 A
- EP 0726310 A
- WO 2006119280 A
- US 2002173016 A
- WO 2008075072 A
- WO 2005108620 A
- US 4318846 A
- WO 9937717 A
- US 6399392 B
- US 7179590 B
- WO 2006003439 A
- WO 9928500 A
- WO 2004033726 A
- WO 9966071 A
- US 5565339 A
- US 5677152 A
- WO 02088387 A

### Non-patent literature cited in the description

- **KAJUWARA et al.** *Pharmaceutical Research,* 1999, vol. 16, 9
- **BECKET et al.** *Reactive & Functional Polymers,* 2004, vol. 60, 183-193
- **DAVIDSON ; SUN.** *Pharm. Res.,* 2001, vol. 18, 474-79
- **RIISOM et al.** *JAOCA,* 1980, vol. 57, 354-359